# FASCICULE DE BREVET EUROPEEN

(11) **EP 2 240 505 B1**
(45) Date de publication et mention de la délivrance du brevet: **21.08.2013**
(21) Numéro de dépôt: 08873330.8
(22) Date de dépôt: 17.12.2008
(51) Int. Cl.: A61K 38/05, A61K 38/06, A61K 38/07, A61K 38/08, A61Q 19/00, A61K 8/64, C07K 5/06, C07K 5/10, C07K 5/08, A61Q 19/08

(54) **PEPTIDE ACTIVATEUR DE LA SYNTHESE DES AQUAPORINES**
PEPTID ZUR AKTIVIERUNG DER AQUAPORINSYNTHESE
PEPTIDE FOR ACTIVATING AQUAPORIN SYNTHESIS

(30) Priorité: 21.12.2007 FR 0708977
(43) Date de publication de la demande: 20.10.2010
(73) Titulaire: Société d'Extraction des Principes Actifs SA (Vincience), 06410 Biot (FR)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2008/001759
(87) Numéro de publication internationale: WO 2009/112645

(56) Documents cités:
- WO-A-92/03147
- FR-A- 2 831 058
- JP-A- 8 245 694
- DATABASE REGISTRY American Chemical Society; Peptide Pro-Ala. Arg (RN no: 480543-38-2) 23 janvier 2003 (2003-01-23), XP002493259
- DUMAS M ET AL: "Hydrating skin by stimulating biosynthesis of aquaporins" JOURNAL OF DRUGS IN DERMATOLOGY, NEW YORK, vol. Supplement, no. 6, 1 juin 2006 (2006-06-01), pages 20-24, XP002488654

## Description

La présente invention se situe dans le domaine cosmétique et pharmaceutique, et plus particulièrement dans le domaine de la dermatologie. La présente invention concerne un peptide capable d'activer la synthèse de protéines de la famille des aquaporines, de formule générale (I) : R₁-(AA)ₙ-X₁-X₂-X₃-Pro-X₄-X₅-X₇-(AA)ₚ-R₂.

La présente invention concerne également une composition cosmétique, nutraceutique ou pharmaceutique, et notamment dermatologique, comprenant au moins un peptide de formule générale (I), en tant que principe actif, seul ou en association avec au moins un autre principe actif. L'invention est également relative à l'utilisation de ce nouveau principe actif, par voie orale ou topique, dans une composition cosmétique ou nutraceutique, destinée à améliorer l'hydratation et la fonction barrière de l'épiderme, et à stimuler la régénération cutanée. L'invention est également relative à l'utilisation de ce nouveau principe actif, par voie orale ou topique, pour la réalisation d'une composition pharmaceutique, et notamment dermatologique, destinée à réguler et/ou stimuler l'activité des aquaporines et traiter ainsi les pathologies impliquant une sècheresse pathologique de la peau ou des muqueuses, telles que la xérose ou l'eczéma, les symptômes de bouche sèche ou la sècheresse de l'oeil. L'invention porte encore sur un procédé de traitement cosmétique destiné à prévenir ou à lutter contre la sècheresse de la peau et des muqueuses, et les manifestations du vieillissement cutané, selon lequel on applique une quantité efficace de peptide capable d'activer la synthèse des aquaporines, ou une composition le contenant, sur les zones à traiter.

La peau est un organe vital composé de plusieurs couches (derme, couches prolifératives et *stratum corneum*) qui recouvre toute la surface du corps et assure essentiellement une fonction de barrière vis-à-vis du milieu extérieur. Cette fonction barrière repose en particulier sur la qualité de l'épiderme qui dépend notamment de la l'état du *stratum corneum* et de l'équilibre entre la prolifération et la différenciation des kératinocytes épidermiques. La qualité et le bon fonctionnement de la peau sont étroitement liés à la teneur en eau des différentes couches de l'épiderme. Ainsi, dans un épiderme normal, les couches prolifératives contiennent environ 70 % d'eau, alors que le *stratum corneum* en contient seulement 10 à 15 %.

L'hydratation du *stratum corneum* est la résultante de trois facteurs : l'approvisionnement en eau à partir du derme, la perte en eau vers le milieu extérieur et la capacité du *stratum corneum* à fixer les molécules d'eau.

La régulation de la distribution en eau se fait par des hormones (aldostérone, hormones sexuelles), le pH ou les variations osmotiques. Les membranes cellulaires sont de nature hydrophobes et donc peu perméables à l'eau, mais il existe des canaux hydriques, sortes de pores facilitant le passage de l'eau et de certains solutés.

Les aquaporines sont une classe de protéines transmembranaires transporteurs d'eau et de petites molécules en solution, telles que le glycérol et l'urée, qui facilitent le transport de l'eau dans les épithéliums et les endothéliums.

La fonction déterminante des aquaporines dans les épithéliums impliqués dans le transport de l'eau ou des solutés, tels que le rein, a été bien étudiée (Deen et al., 1994). De même, les aquaporines ont été rapidement identifiées dans les glandes exocrines productrices de la salive ou des pleurs. Toutefois, la découverte d'aquaporines de type 3, ou AQP3, dans l'épiderme humain, et plus précisément dans la membrane plasmique des kératinocytes des couches prolifératives de l'épiderme (SOUGRAT R. et al., J. Invest. Dermatol., 2002), a mis en lumière l'importance des flux hydriques régulés dans la peau. Les AQP3 sont capables de transporter l'eau et le glycérol, ce dernier jouant un rôle important dans la constitution du film hydrolipidique de surface ainsi que dans le maintien de la souplesse et des qualités sensorielles du *stratum corneum.*

L'importance des AQP3 a été démontrée chez la souris. En effet, l'inactivation du gène provoque l'apparition de multiples déficiences de la peau, telles qu'un faible taux d'hydratation, une fonction barrière inefficace, un temps de régénération tissulaire augmenté et une diminution de l'élasticité (Ma T. et al., J. Mol. Biol., 2002). On a découvert depuis que l'aquaporine 3 est exprimée dans les fibroblastes dermiques où elle intervient dans la migration de ces cellules lors de la régénération des plaies (Cao C. et al., Biochem J., 2006). D'autre part, les aquaporines jouent un rôle dans la fonction barrière en régulant positivement l'établissement des liaisons et communications cellulaires de type tight junction (KAwedia J. et al., PNAS 104(9), 2007).

L'hydratation et la teneur en AQP3 des kératinocytes sont étroitement liées. Ainsi, l'augmentation d'AQP3 dans la peau provoque une meilleure hydratation de l'épiderme (Dumas M., J. Drugs Dermatol., Jun6, 2007).

Les pertes cutanées en eau peuvent avoir plusieurs origines, héréditaires, acquises ou liées à l'environnement. Dans un environnement très sec, les pertes en eau par évaporation à partir du *stratum corneum* sont significatives et peuvent excéder le taux de remplacement par diffusion transcellulaire.

Au cours du vieillissement cutané, la peau devient sèche. Ainsi, il est très souvent constaté chez les sujets âgés, et notamment de plus de 50 ans, la manifestation d'une xérose ou d'une sècheresse des muqueuses, liée à une moindre sécrétion de sébum, à des modifications hormonales ou à des ralentissements du flux hydrique à travers l'épiderme. La peau est alors le siège de démangeaisons et de tiraillements, deux symptômes caractéristiques d'une peau sèche. Parmi les pathologies acquises se traduisant par une sècheresse de la peau, on peut citer la xérose induite par la photo-chimiothérapie et l'eczéma. Parmi les pathologies acquises provoquant une sècheresse de la bouche, ou xérostomie, on peut citer le syndrome de Sjogren ou la radiothérapie du cou. Des sécheresses pathologiques peuvent également affecter d'autres zones muqueuses de l'organisme, parmi lesquelles on peut citer la muqueuse vaginale et la muqueuse oculaire.

Une première alternative de traitement des peaux sèches consiste à administrer par voie topique des produits destinés à restaurer la barrière cutanée, tels que des agents humectants capables de fixer l'eau, parmi lesquels on peut citer l'urée et l'acide lactique qui entrent dans la composition du NMF (Natural Moisturizer Factor ; dérivés protéolytiques de la filaggrine), des agents filmogènes destinés à retenir l'eau, ou encore des agents capables de reconstruire la barrière cutanée (squalènes, céramides acides gras). Toutefois, ces produits ont une action superficielle qui ne corrige pas les défauts biologiques d'une peau souffrant de déshydratation chronique.

Dans ce contexte, les propriétés particulières des aquaporines en font des cibles biologiques possibles pour améliorer l'hydratation de la peau et diminuer les signes de sècheresse cutanée. Ainsi, le brevet FR 2 801 504 décrit l'augmentation d'AQP3 dans la peau par un extrait de plantes *Ajuga turkestanica,* et a permis d'améliorer l'hydratation de la peau. Un extrait de grenade a, part ailleurs, été décrit comme principe actif, utilisable par voies orale et topique, pour stimuler l'activité des aquaporines et réguler les mouvements d'eau et de glycérol dans les tissus (FR 2 874 502). Toutefois, à ce jour, aucun peptide capable d'activer les aquaporines, tel que divulgué dans la présente invention, n'a encore été décrit.

La présente invention a pour principal objectif de fournir un nouveau principe actif capable de prévenir et de lutter contre la sècheresse de la peau et des muqueuses et les manifestations du vieillissement cutané. Les inventeurs ont en effet mis en évidence une activité biologique utile dans le domaine cosmétique et thérapeutique, notamment dermatologique, de peptides capables d'activer la synthèse des aquaporines.

Il a en particulier été mis en évidence que ces peptides, lorsqu'ils sont appliqués sur la peau, améliorent l'hydratation et la fonction barrière de l'épiderme et des muqueuses, et stimulent la régénération cutanée. Ces propriétés ont été démontrées par une protection du tissu cutané et une diminution de l'apoptose à la suite d'un stress hydrique.

On entend par « principe actif capable de prévenir et de lutter contre la sècheresse de la peau et des muqueuses et les manifestations du vieillissement cutané», toute substance capable d'améliorer l'hydratation et la fonction barrière de l'épiderme et des muqueuses, ou de diminuer l'apoptose dans des cellules ou des tissus soumis à un stress hydrique.

On entend par « application topique », le fait d'appliquer ou d'étaler le principe actif selon l'invention, ou une composition le contenant, à la surface de la peau ou d'une muqueuse.

On entend par « cosmétiquement ou dermatologiquement acceptable », que le principe actif selon l'invention, ou une composition le contenant, est approprié pour entrer en contact avec la peau ou une muqueuse sans provoquer de réactions de toxicité ou d'intolérance.

On entend par « nutraceutique », une méthode de soin de la peau, qui implique l'administration de principes actifs par des voies autres que la voie topique, à des sujets en bonne santé qui veulent améliorer ou entretenir leur apparence physique.

Ainsi, l'invention a pour objet premier un peptide capable d'activer la synthèse des aquaporines, en tant que principe actif, seul ou en association avec au moins un autre principe actif.

On entend par « principe actif capable d'activer la synthèse des aquaporines », tout peptide ou dérivé biologiquement actif capable d'augmenter l'activité des aquaporines, soit par l'augmentation de la synthèse protéique de l'aquaporine (par modulation directe ou indirecte de l'expression génique de l'aquaporine), soit par l'augmentation de l'activité biochimique de l'aquaporine, soit par d'autres processus biologiques tels que la stabilisation de la protéine aquaporine ou encore la stabilisation des transcrits d'ARN messager.

Préférentiellement, selon la présente invention, ledit peptide capable d'activer l'aquaporine, ou son dérivé biologiquement actif, est un peptide dont le nombre d'acides aminés est compris entre 2 et 15.

Préférentiellement, selon la présente invention, l'aquaporine sera l'aquaporine 3, ou AQP3, présente dans les membranes des kératinocytes.

Par l'expression « biologiquement actif », on entend « qui possède une activité *in vivo* ou *in vitro* caractéristique de l'activité du principe actif selon l'invention ».

Selon une méthode particulièrement avantageuse de réalisation de l'invention, le peptide possède une séquence qui répond en tout ou en partie à la formule générale (I)

R₁-(AA)ₙ-X₁-X₂-X₃-Pro-X₄-X₅-X₇-(AA)ₚ-R₂

Dans laquelle,
X₁ est la sérine ou aucun acide aminé,
X₂ est la méthionine ou la leucine ou aucun acide aminé,
X₃ est l'asparagine ou la glutamine ou aucun acide aminé,
X₄ est l'alanine ou la glycine,
X₅ est l'arginine ou la lysine ou aucun acide aminé,
X₆ est la sérine, l'acide aspartique ou aucun acide aminé
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄.

Selon une méthode de réalisation de l'invention tout particulièrement préférée, le peptide biologiquement actif est de séquence :
(SEQ ID n°1) Ser-Met-Asn-Pro-Gly-Arg
(SEQ ID n°2) Gln-Pro-Ala-Lys-Ser
(SEQ ID n°3) Asn-Pro-Ala-Arg-Asp
(SEQ ID n°4) Asn-Pro-Ala-Arg
(SEQ ID n°5) Asn-Pro-Ala-Arg-NH₂
(SEQ ID n°6) Asn-Pro-Ala
(SEQ ID n°7) Asn-Pro-Ala-NH₂
(SEQ ID n°8) Pro-Ala-Arg
(SEQ ID n°9) Pro-Ala-Arg-NH₂
(SEQ ID n°10) Leu-Asn-Pro-Ala

Selon un mode de réalisation particulièrement intéressant, le peptide biologiquement actif correspond à la séquence SEQ ID n°4.

Selon un autre mode de réalisation particulièrement intéressant, le peptide biologiquement actif correspond à la séquence SEQ ID n°5.

Dans l'invention, le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées.

Par « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une composition telle que définie précédemment, caractérisée par le fait que le peptide de séquence SEQ ID n°1 à SEQ ID n°10 est sous forme protégée ou non. On peut utiliser une protection basée sur une substitution sur l'extrémité amino-terminale par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. Il est également possible de protéger les deux extrémités du peptide.

Les dérivés de peptides concernent aussi les acides aminés et les peptides reliés entre eux par une liaison pseudo-peptidique. On entend par « liaison pseudo-peptidique », tous les types de liaisons susceptibles de remplacer les liaisons peptidiques « classiques ».

Dans le domaine des acides aminés, les molécules une géométrie telle, qu'elles peuvent théoriquement se présenter sous la forme d'isomères optiques différents. Il existe ainsi une conformation moléculaire de l'acide aminé (AA) qui dévie à droite le plan de polarisation de la lumière (conformation dextrogyre ou D-aa), et une conformation moléculaire de l'acide aminé (aa) qui dévie à gauche le plan de polarisation de la lumière (conformation lévogyre ou L-aa). Les acides aminés naturels sont toujours de conformation lévogyre, en conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre que lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D- ou DL-.

Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut également être obtenu par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement ou partiellement aux peptides de formule générale (I).

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides de formule générale I selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi, le peptide selon l'invention peut être d'origine naturelle ou synthétique. Préférentiellement selon l'invention, le peptide est obtenu par synthèse chimique.

Selon l'invention, le principe actif peut être un peptide unique, un mélange de peptides ou de dérivés peptidiques et/ou constitués de dérivés d'acides aminés.

Selon un mode de réalisation avantageux de l'invention, le principe actif selon l'invention est préalablement solubilisé dans un ou plusieurs solvants cosmétiquement ou pharmaceutiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

L'invention a pour second objet une composition cosmétique, nutraceutique ou pharmaceutique, et notamment dermatologique, comprenant, dans un milieu physiologiquement adapté, une quantité efficace de peptide capable d'activer la synthèse des aquaporines, en tant que principe actif, seul ou en association avec au moins un autre principe actif, tel que défini précédemment.

Il est bien évident que l'invention s'adresse aux mammifères en général, et plus particulièrement aux êtres humains.

La quantité efficace de principe actif correspond à la quantité nécessaire pour obtenir le résultat recherché, à savoir, améliorer l'hydratation et la fonction barrière de l'épiderme, et/ou stimuler la régénération cutanée, et/ou réguler ou stimuler l'activité des aquaporines, et/ou prévenir ou lutter contre la sècheresse de la peau et des muqueuses, et les manifestations du vieillissement cutané.

Selon un mode de réalisation avantageux de l'invention, le principe actif selon l'invention est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

La composition utilisable selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. Ainsi, le principe actif selon l'invention pourra avantageusement être utilisé dans les soins antipelliculaires du cuir chevelu.

Elle peut également se présenter sous forme de teinture ou de mascara à appliquer au pinceau ou au peigne, en particulier sur les cils, les sourcils ou les cheveux.

Il est bien entendu que le principe actif selon l'invention peut être utilisé seul ou bien en association avec au moins un autre principe actif, dans une composition cosmétique ou nutraceutique, ou pour la préparation d'une composition pharmaceutique et/ou dermatologique.

Avantageusement, les compositions utilisables selon l'invention contiennent en outre divers agents actifs hydratants, destinés, notamment, à la prévention et/ou au traitement des désordres liés au vieillissement. Ainsi, la composition selon l'invention peut associer du glycérol, en tant qu'agent actif adjuvant, à l'agent actif décrit dans la présente invention. Cette association est particulièrement avantageuse et procure une efficacité d'hydratation optimale, comparée à l'utilisation du glycérol seul.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Ces compositions doivent donc contenir un milieu cosmétiquement et/ou dermatologiquement acceptable, c'est-à-dire compatible avec la peau et les phanères, et couvrent toutes .les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres et adaptées à une application sur la peau, les lèvres et/ou les phanères. Ces compositions comprennent les excipients nécessaires à leur formulation, tels que solvants, épaississants, diluants, tensioactifs, anti-oxydants, colorants, conservateurs, parfums.

Selon une autre forme de l'invention, les compositions seront appropriées à une administration orale pour un usage pharmaceutique ou nutraceutique. Ainsi, les compositions pourront notamment se présenter sous forme de comprimés, capsules, gélules, pâtes à mâcher, poudres à consommer telles quelles ou à mélanger extemporanément avec un liquide, sirops, gels, et toute autre forme connue de l'homme du métier. Elles contiendront des excipients de formulation appropriés, tels que colorants, édulcorants, aromatisants, agents de charge, liants, conservateurs.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids, et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

L'invention a pour troisième objet l'utilisation du principe actif dans des compositions cosmétiques ou nutraceutiques, ou pour la préparation de compositions pharmaceutiques.

Ainsi, le principe actif, grâce à ses propriétés particulières, pourra être utilisé dans une composition cosmétique ou nutraceutique destinée à améliorer l'hydratation et la fonction barrière de l'épiderme ou destinée à prévenir ou à traiter la sècheresse cutanée.

D'autre part, le principe actif selon l'invention pourra être utilisé avantageusement dans une composition cosmétique ou nutraceutique destinée à lutter de manière préventive et/ou curative contre les manifestations du vieillissement cutané, et plus spécifiquement, afin de lutter contre et/ou de prévenir le vieillissement photo-induit (photo-vieillissement). Par manifestations cutanées du vieillissement, on entend toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement du derme ou toute autre dégradation interne de la peau consécutive à une exposition aux rayonnements ultraviolets (UV). Ainsi, le principe actif selon l'invention, ou la composition le contenant, permettra de lutter, en particulier, contre la perte d'efficacité de la fonction barrière de la peau et permettra de stimuler la régénération du derme et de l'épiderme.

Selon un autre aspect de l'invention, le principe actif selon l'invention pourra être utilisé avantageusement dans une composition cosmétique ou nutraceutique destinée à protéger la peau et les muqueuses contre tous types d'agressions extérieures.

On entend par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette. La sècheresse de l'atmosphère est également une cause importante de dessèchement cutané.

Les agressions que subissent la peau et les cheveux sont dues, par exemple, à un déséquilibre du gradient électrochimique à travers la membrane cellulaire, ce qui peut aboutir à des variations importantes de la pression osmotique et cela peut avoir pour conséquence des chocs osmotiques et donc la lyse des cellules.

Or les inventeurs ont démontré, d'une manière surprenante, que le principe actif selon l'invention protège les cellules contre ces chocs osmotiques.

La peau peut également être agressée par des traitements tels que le rasage. Avantageusement, l'invention a pour objet l'utilisation dans une composition cosmétique, d'une quantité efficace de principe actif tel que décrit précédemment, le principe actif, ou la composition le contenant, étant destiné à prévenir ou à traiter les dommages causés à la peau par le rasage.

Selon encore un autre aspect de l'invention, le principe actif pourra être utilisé avantageusement dans une composition cosmétique ou nutraceutique, ou pour préparer une composition pharmaceutique, destinée à stimuler la régénération cutanée.

L'invention a également pour objet l'utilisation dans une composition cosmétique, ou pour la préparation d'une composition pharmaceutique, d'une quantité efficace de principe actif tel que décrit précédemment, le principe actif, ou la composition le contenant, étant destiné à prévenir les dommages causés à la peau par une exposition au soleil ou à un environnement desséchant.

L'invention consiste encore en l'utilisation du principe actif selon l'invention, pour la préparation d'une composition pharmaceutique destinée à réguler et/ou stimuler l'activité des aquaporines et prévenir ou traiter ainsi les pathologies provoquées par les dysfonctionnements des aquaporines de la peau et des muqueuses, l'eczéma, la xérose, les dermatites atopiques, les sécheresses buccales, oculaires ou vaginales.

L'invention consiste encore en un procédé de traitement cosmétique destiné à améliorer l'aspect de la peau et prévenir ou à lutter contre la sècheresse de la peau et des muqueuses, selon lequel on applique une quantité efficace d'un peptide selon l'invention capable d'activer l'aquaporine ou une composition le contenant sur les zones à traiter.

L'invention consiste encore en un procédé de traitement cosmétique destiné à prévenir et/ou à lutter contre les signes cutanés du vieillissement et/ou du photo-vieillissement, selon lequel on applique une quantité efficace d'un peptide selon l'invention, capable d'activer l'aquaporine, ou une composition le contenant, sur les zones à traiter.

Des modes de réalisation particuliers de ce procédé de traitement cosmétique résultent également de la description précédente. D'autres avantages et caractéristiques de l'invention apparaîtront mieux à la lecture des exemples.

### Exemple 1 : Evaluation de l'effet protecteur du peptide SEQ ID n°4 vis-à-vis d'un choc osmotique

Le but de cette étude est de déterminer l'effet protecteur du peptide SEQ ID n°4 vis-à-vis de kératinocytes humains normaux soumis à un choc osmotique provoqué par le sorbitol. L'évaluation de l'état cellulaire s'effectue ensuite par un test de viabilité au MTT.

### Protocole :

Des kératinocytes humains normaux en culture ont été mis en présence du peptide SEQ ID n°4 à 0,5 %, 1 % et 3 % à partir d'une solution mère à 50 ppm, 24 heures avant et pendant le choc osmotique. Les conditions de culture hypertoniques sont réalisées en ajoutant du sorbitol à 250 mM au milieu de culture pendant 24 heures. Des contrôles non traités sont réalisés.

Des tests de viabilité cellulaire ont ensuite été réalisés par la technique au MTT. L'agent MTT (3-[4, 5-diméthylthiazol-2-yl]-2, 5-diphényltétrazolium bromide) est utilisé pour évaluer la viabilité cellulaire. Les kératinocytes sont incubés dans une solution contenant 0,1 mg/ml de MTT (3-[4, 5-diméthylthiazol-2-yl]-2, 5-diphényltétrazolium bromide). Ce composé est absorbé par les cellules vivantes puis métabolisé par les enzymes mitochondriales (succinate déshydrogénase) en un composé bleu violet, le formazan, qui se présente sous forme de cristaux violets insolubles en milieu aqueux.

Les cristaux de formazan sont solubilisés dans du DMSO. Ils produisent une densité optique (D.O.) proportionnelle au nombre de cellules vivantes présentes dans l'échantillon. Des mesures de densité optique à 540 nm sont ensuite réalisées. La D.O. est alors directement proportionnelle à l'activité enzymatique ainsi qu'au nombre de cellules vivantes.

### Résultats :

Les résultats obtenus montrent une augmentation de la viabilité, dose-dépendante, des cellules soumises au choc osmotique lorsque les cellules sont traitées avec le peptide SEQ ID n°4, en comparaison avec les cellules non traitées. L'augmentation de viabilité est de 12 % lorsque les cellules sont traitées avec le peptide SEQ ID n°4 à 3 %,

### Conclusions :

Le peptide SEQ ID n°4 protège efficacement les kératinocytes humains normaux soumis à un choc osmotique.

### Exemple 2 : Evaluation de l'effet protecteur du peptide SEQ ID n°5 vis-à-vis d'une déshydratation cutanée induite

Le but de cette étude est de déterminer l'effet protecteur du peptide SEQ ID n°5 vis-à-vis de cultures d'épiderme *ex vivo* soumises à un stress par une déshydratation cutanée induite.

### Protocole :

Des biopsies de peau humaine sont débarrassées de leur couche cornée par arrachage à l'aide de ruban adhésif (tape stripping) puis maintenues en culture *ex vivo,* traitées avec une solution à 1 % d'une solution mère à 50 ppm de peptide SEQ ID n°5 pendant 24 heures. L'absence de couche cornée induit une déshydratation importante. Des coupes et des colorations histologiques hématoxyline-éosine (H&E) permettent d'évaluer la qualité des structures cutanées.

### Résultats :

L'observation des coupes de peau montrent une nette diminution des signes de stress cellulaire et une meilleure préservation des structures cutanées dans les biopsies de peau traitées par le peptide SEQ ID n°5, comparées aux biopsies de peau non traitées. D'autre part, l'aspect des kératinocytes de l'assise basale montre un effet régénérant du peptide SEQ ID n°5.

### Conclusions :

Le peptide SEQ ID n°5 protège efficacement la peau du stress induit par la déshydratation. D'autre part, le peptide SEQ ID n°5 permet une régénération de l'épiderme.

### Exemple 3: Mise en évidence de l'effet activateur du peptide SEQ ID n°5 sur l'expression des claudines et de la kératine K10

Le but de cette étude est de déterminer l'influence du peptide SEQ ID n°5 sur l'expression des claudines et de la kératine K10. Les claudines sont les principales protéines transmembranaires des structures d'adhésion intercellulaires nommées tight-junction, qui jouent un rôle dans la communication cellulaire et la cohésion épidermique. La kératine K10 est une kératine spécifique des couches épidermiques différenciées (*stratum granulosum*) et intervient dans la fonction barrière de la peau. La quantité de protéine a été évaluée par immunofluorescence sur des coupes de peau humaine.

Protocole : Des échantillons de peau humaine sont mis en culture à l'interface air/liquide. Une solution à 1 %,d'une solution mère à 50 ppm de peptide SEQ ID n°5 est appliquée topiquement, puis les échantillons sont incubés durant 24 heures.

Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes.

L'immunomarquage de la Claudin-1 est réalisé à l'aide d'un anticorps spécifique de la claudin-1 (anticorps claudin-1: rabbit polyclonal, ref: Ab15098, Abcam, dilution 1/200^{ème}), puis d'un anticorps secondaire, couplé à un marqueur fluorescent (alexa Fluor 488 donkey anti-rabbit IgG, A21206, Molecular Probes, 1/1000^{ème}).

L'immunomarquage de la Kératine 10 est réalisé à l'aide d'un anticorps spécifique de la kératine 10 (anticorps K10: mouse monoclonal, ref: LHP1, Novocastra, dilution 1150^{ème}), puis d'un anticorps secondaire, couplé à un marqueur fluorescent (alexa Fluor 488 donkey anti-mouse IgG, A21202, Molecular Probes, 1/1000^{ème}).

Les coupes de peau sont alors examinées au microscope à Epi-fluorescence (Nikon Eclipse E600 microscope).

Résultats : Les observations microscopiques montrent une fluorescence plus forte dans les peaux traitées par le peptide SEQ ID n°5 à 1 %, en particulier les couches supérieures de l'épiderme aussi bien pour la protéine claudin-1 que pour la kératine K10.

Conclusions : Le peptide SEQ ID n°5, à la concentration de 0,05 ppm, stimule fortement l'expression des claudines, en particulier dans les couches supérieures de l'épiderme. De même, le peptide SEQ ID n°5, à la concentration de 0,05 ppm, stimule fortement l'expression de la kératine K10, et plus généralement les fonctions barrières de l'épiderme.

### Exemple 4 : Mise en évidence de l'effet stimulant du peptide SEQ ID n°5 sur l'expression d'aquaporines

Le but de cette étude est de déterminer l'influence du peptide SEQ ID n°5 sur l'expression de l'aquaporine 3 dans des échantillons de peau *ex vivo.*

Protocole : Des échantillons de peau humaine sont mis en culture à l'interface air/liquide. Une solution à 1 %, d'une solution mère à 50 ppm de peptide SEQ ID n°4 est appliquée topiquement, puis les échantillons sont incubés durant 24 heures ou 48 heures.

Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes. L'immunomarquage est réalisé à l'aide d'un anticorps polyclonal spécifique de l'aquaporine 3 (Anti-aquaporin 3 (C-18): goat polyclonal, sc-9885, Santa Cruz, dilution 1/100^{ème}), puis d'un anticorps secondaire, couplé à un marqueur fluorescent (Alexa-fluor 488 donkey anti-goat Ig G, Molecular Probes, dilution 1/1000^{ème}). Les coupes de peau sont alors examinées au microscope à Epi-fluorescence (Nikon Eclipse E600 microscope).

Résultats : Les observations microscopiques montrent une fluorescence plus forte dans les peaux traitées par le peptide SEQ ID n°4 à 1 %, en particulier dans les couches supérieures de l'épiderme par rapport au contrôle non traité.

Conclusions : Le peptide SEQ ID n°4, à la concentration de 0,05 ppm, stimule l'expression de l'aquaporine 3, en particulier dans les couches supérieures de l'épiderme.

### Exemple 5: Préparation de compositions

### 1 - Crème de jour

| ***Noms commerciaux*** | ***Noms INCI*** | ***% massique*** |
|---|---|---|
| ***PHASE A*** | | |
| MONTANOV 68 | Cetearyl Alcohol (and) Cetearyl Glucoside | 5,00 |
| HUILE DE JOJOBA | Simmondsia Chinensis (Jojoba) Seed Oil | 3,00 |
| HUILE DE VASELINE | Paraffinum Liquidum (Mineral Oil) | 2,00 |
| SQUALANE | Squalane | 3,00 |
| CERAPHYL 368 | Ethylhexyl palmitate | 4,00 |
| CERAPHYL 41 | C12-C15 Alkyl Lactate | 3,00 |
| RAPITHIX A-60 | Sodium polyacrylate (and) Hydrogenated Polydecene (and) Trideceth -6 | 0,30 |

| ***PHASE B*** | | |
|---|---|---|
| GLYCERINE | Glycerin | 5,00 |
| ALLANTOÏNE | Allantoin | 0,10 |
| EAU DEMINERALISEE | Aqua (Water) | qsp 100 |

| ***PHASE C*** | | |
|---|---|---|
| ROKONSAL MEP | Phenoxyethanol (and) Methylparaben (and) Ethylparaben (and) Propylparaben | 0,50 |

| ***PHASE D*** | | |
|---|---|---|
| PEPTIDE SEQ ID n° 5 | | 5 ppm |

| ***PHASE E*** | | |
|---|---|---|
| PARFUM | Parfum (Fragrance) | qsp |

### Mode Opératoire :

Peser les ingrédients de la phase grasse et chauffer à 70°C sous agitation. Préparer la phase B et la chauffer à 70°C. Emulsionner la phase A dans la phase B. Ajouter la phase D vers 50°C sous agitation. En dessous de 40°C, additionner le principe actif (Phase D). Parfumer et refroidir jusqu'à température ambiante.

### 2 - Crème hydratante W/O

| ***Noms commerciaux*** | ***Noms INCI*** | ***% massique*** |
|---|---|---|
| ***PHASE A*** | | |
| ARLACEL P 135 | PEG-30 Dipolyhydroxystearate Isononanoate | 2,00 |
| CERAPHYL 375 | Isostearyl Neopentanoate | 3.00 |
| PANALANE L-14E | Hydrogenated Polyisobutene | 3,00 |
| CERAPHYL ODS | Octyldodecyl Stearate | 9,00 |
| CERAPHYL 368 | Ethylhexyl Palmitate | 3,00 |

| ***PHASE B*** | | |
|---|---|---|
| EAU DEMINERALISEE | Aqua (Water) | qsp 100 |
| ATLAS G-2330 | Sorbeth-30 | 4,00 |
| SULFATE DE MAGNESIUM 7 H2O | Magnesium Sulfate | 0,70 |
| PEPTIDE SEQ ID n° 5 | | 3 ppm |

| ***PHASE C*** | | |
|---|---|---|
| LIQUAPAR OPTIMA | Phenoxyethanol (and) Methylparaben (and) Isopropylparaben (and) Isobutylparaben (and) Butylparaben | 0,50 |

| ***PHASE D*** | | |
|---|---|---|
| PARFUM | Parfum (Fragrance) | qsp |

### Mode Opératoire :

Peser la phase A et chauffer à 75°C sous agitation. Préparer la phase B et la chauffer à 75°C. Emulsionner la phase B dans la phase A sous forte agitation rotor-stator.

Homogénéiser quelques minutes. Refroidir brutalement à l'aide d'un bain d'eau glacée sous vive agitation. Ajouter la phase C aux environs de 50°C et additionner le parfum (phase D) à 40°C. Poursuivre le refroidissement jusqu'à température ambiante.

### 3 - Lotion hydratante

| ***Noms commerciaux*** | ***Noms INCI*** | ***% massique*** |
|---|---|---|
| EAU DEMINERALISEE | Aqua (Water) | qsp 100 |
| GLYCERINE | Glycerin | 2,00 |
| PROPYLENE GLYCOL | Propylene Glycol | 2,00 |
| GLUCAM E-10 | Methyl Gluceth -10 | 1,00 |
| NEOSORB | Sorbitol | 5,00 |
| ALLANTOINE | Allantoin | 0,10 |
| ROKONSAL BSB | Benzoic Acid (and) Sorbic Acid (and) Benzyl Alcohol | 0,30 |
| PEPTIDE SEQ ID n° 4 | | **1 ppm** |
| PARFUM hydrosoluble | Parfum (Fragrance) | qsp |

### Mode Opératoire :

Incorporer les ingrédients un à un à la quantité d'eau nécessaire et agiter jusqu'à parfaite dissolution. Réajuster le pH aux environs de 5,5 si nécessaire. Incorporer l'actif en fin de formulation. Parfumer avec un parfum hydrosoluble sous faible agitation.

## Revendications

1. Peptide capable d'activer la synthèse de protéines de la famille des aquaporines, **caractérisé en ce qu'**il correspond à une des séquences suivantes :
(SEQ ID n°1) Ser-Met-Asn-Pro-Gly-Arg
(SEQ ID n°2) Gln-Pro-Ala-Lys-Ser
(SEQ ID n°3) Asn-Pro-Ala-Arg-Asp
(SEQ ID n°4) Asn-Pro-Ala-Arg
(SEQ ID n°5) Asn-Pro-Ala-Arg-NH₂
(SEQ ID n°6) Asn-Pro-Ala
(SEQ ID n°7) Asn-Pro-Ala-NH₂
(SEQ ID n°9) Pro-Ala-Arg-NH₂

2. Peptide selon la revendication 1 **caractérisé en ce qu'**il correspond à la séquence SEQ ID n°4.

3. Peptide selon la revendication 1 **caractérisé en ce qu'**il correspond à la séquence SEQ ID n°5.

4. Peptide selon l'une quelconque des revendications précédentes, **caractérisé en ce qu'**il possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

5. Peptide selon l'une quelconque des revendications précédentes, **caractérisé en ce que** les protéines de la famille des aquaporines sont des aquaporines de type 3.

6. Composition cosmétique ou pharmaceutique **caractérisée en ce qu'**elle contient dans un milieu physiologiquement acceptable, en tant que principe actif, une quantité efficace d'un peptide capable d'activer la synthèse de protéines de la famille des aquaporines et correspondant à une des séquences suivantes :
(SEQ ID n°1) Ser-Met-Asn-Pro-Gly-Arg
(SEQ ID n°2) Gln-Pro-Ala-Lys-Ser
(SEQ ID n°3) Asn-Pro-Ala-Arg-Asp
(SEQ ID n°4) Asn-Pro-Ala-Arg
(SEQ ID n°5) Asn-Pro-Ala-Arg-NH₂
(SEQ ID n°6) Asn-Pro-Ala
(SEQ ID n°7) Asn-Pro-Ala-NH₂
(SEQ ID n°8) Pro-Ala-Arg
(SEQ ID n°9) Pro-Ala-Arg-NH₂
préalablement solubilisé dans un ou plusieurs solvants cosmétiquement ou pharmaceutiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

7. Composition cosmétique ou pharmaceutique selon la revendication 6, **caractérisée en ce que** ledit peptide est présent à une concentration comprise entre 0,0005 et 500 ppm environ, et préférentiellement à une concentration comprise entre 0,01 et 5 ppm.

8. Composition selon l'une des revendications 6 ou 7, **caractérisée en ce qu'**elle contient en outre du glycérol en tant qu'agent hydratant.

9. Utilisation d'une quantité efficace de peptide, seul ou en association avec un autre principe actif, pour la préparation d'une composition cosmétique ou nutraceutique destinée à améliorer l'hydratation et la fonction barrière de l'épiderme, ledit peptide étant capable d'activer la synthèse de protéines de la famille des aquaporines et étant de formule générale (I)
R₁-(AA)ₙ-X₁-X₂-X₃-Pro-X₄-X₅-X₆-(AA)p-R₂
dans laquelle,
X₁ est la sérine ou aucun acide aminé,
X₂ est la méthionine ou la leucine ou aucun acide aminé,
X₃ est l'asparagine ou la glutamine ou aucun acide aminé,
X₄ est l'alanine ou la glycine,
X₅ est l'arginine ou la lysine ou aucun acide aminé,
X₆ est la sérine, l'acide aspartique ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 2 à 15 résidus d'acides aminés,
ladite séquence de formule générale (I) pouvant comprendre des substitutions des acides aminés X₁ à X₆ par d'autres acides aminés chimiquement équivalents.

10. Utilisation d'une quantité efficace de peptide, tel que défini dans la revendication 9, en tant que principe actif, seul ou en association avec un autre principe actif, pour la préparation d'une composition cosmétique ou nutraceutique destinée à prévenir ou à traiter la sècheresse cutanée.

11. Utilisation d'une quantité efficace de peptide, tel que défini dans la revendication 9, en tant que principe actif, seul ou en association avec un autre principe actif, pour la préparation d'une composition cosmétique ou nutraceutique destinée à protéger la peau et les muqueuses des agressions extérieures.

12. Utilisation d'une quantité efficace de peptide, tel que défini dans la revendication 9, en tant que principe actif, seul ou en association avec un autre principe actif, pour la préparation d'une composition cosmétique ou nutraceutique ou pharmaceutique destinée à stimuler la régénération cutanée.

13. Utilisation d'une quantité efficace de peptide, tel que défini dans la revendication 9, en tant que principe actif, seul ou en association avec un autre principe actif, pour la préparation d'une composition cosmétique ou nutraceutique destinée à prévenir ou à traiter les signes cutanés du vieillissement et/ou du photo-vieillissement.

14. Utilisation d'une quantité efficace de peptide, tel que défini dans la revendication 9, en tant que principe actif, seul ou en association avec un autre principe actif, pour préparer une composition pharmaceutique destinée à traiter les états pathologiques liés à la sècheresse de la peau ou des muqueuses.

15. Procédé de traitement cosmétique destiné à améliorer l'aspect de la peau et à prévenir et/ou lutter contre la sècheresse de la peau et des muqueuses, **caractérisé en ce que** l'on applique topiquement sur la peau ou les muqueuses à traiter une composition telle que définie selon l'une quelconque des revendications 6 à 8.

16. Procédé de traitement cosmétique destiné à prévenir et/ou à lutter contre les signes cutanés du vieillissement et/ou du photo-vieillissement, **caractérisé en ce que** l'on applique topiquement sur la peau à traiter une composition telle que définie selon l'une quelconque des revendications 6 à 8.

## Patentansprüche

1. Peptid, das dazu in der Lage ist, die Synthese von Proteinen der Familie der Aquaporine zu aktivieren, **dadurch gekennzeichnet, dass** es einer der folgenden Sequenzen entspricht:
(SEQ ID Nr. 1) Ser-Met-Asn-Pro-Gly-Arg
(SEQ ID Nr. 2) Gln-Pro-Ala-Lys-Ser
(SEQ ID Nr. 3) Asn-Pro-Ala-Arg-Asp
(SEQ ID Nr. 4) Asn-Pro-Ala-Arg
(SEQ ID Nr. 5) Asn-Pro-Ala-Arg-NH₂
(SEQ ID Nr. 6) Asn-Pro-Ala
(SEQ ID Nr. 7) Asn-Pro-Ala-NH₂
(SEQ ID Nr. 9) Pro-Ala-Arg-NH₂

2. Peptid nach Anspruch 1, **dadurch gekennzeichnet, dass** es der Sequenz SEQ ID Nr. 4 entspricht.

3. Peptid nach Anspruch 1, **dadurch gekennzeichnet, dass** es der Sequenz SEQ ID Nr. 5 entspricht.

4. Peptid nach einem der vorhergehenden Ansprüche, **dadurch gekennzeichnet, dass** es mindestens eine funktionelle Gruppe aufweist, die von einer Schutzgruppe geschützt wird, wobei diese Schutzgruppe entweder eine Acylierung oder eine Acetylierung des amino-terminalen Endes ist, oder eine Amidbildung oder eine Veresterung des carboxy-terminalen Endes oder beides ist.

5. Peptid nach einem der vorhergehenden Ansprüche, **dadurch gekennzeichnet, dass** die Proteine der Familie der Aquaporine Aquaporine vom Typ 3 sind.

6. Kosmetische oder pharmazeutische Zusammensetzung, **dadurch gekennzeichnet, dass** sie in einem physiologisch akzeptablen Medium als Wirkstoff eine wirksame Menge eines Peptids enthält, die dazu in der Lage ist, die Synthese von Proteinen der Familie der Aquaporine zu aktivieren, die einer der folgenden Sequenzen entsprechen:
(SEQ ID Nr. 1) Ser-Met-Asn-Pro-Gly-Arg
(SEQ ID Nr. 2) Gln-Pro-Ala-Lys-Ser
(SEQ ID Nr. 3) Asn-Pro-Ala-Arg-Asp
(SEQ ID Nr. 4) Asn-Pro-Ala-Arg
(SEQ ID Nr. 5) Asn-Pro-Ala-Arg-NH₂
(SEQ ID Nr. 6) Asn-Pro-Ala
(SEQ ID Nr. 7) Asn-Pro-AIa-NH₂
(SEQ ID Nr. 8) Pro-Ala-Arg
(SEQ ID Nr. 9) Pro-Ala-Arg-NH₂
zuvor solubilisiert in einem oder in mehreren kosmetisch oder pharmazeutisch akzeptablen Lösemitteln wie z.B. Wasser, Glycerol, Ethanol, Propylenglycol, Butylenglycol, Dipropylenglycol, ethoxylierten oder propoxylierten Diglykolen, cyclischen Polyolen, Vaselin, einem pflanzlichen Öl oder jeder Mischung dieser Lösemittel.

7. Kosmetische oder pharmazeutische Zusammensetzung nach Anspruch 6, **dadurch gekennzeichnet, dass** das Peptid in einer Konzentration zwischen ungefähr 0,0005 und 500 ppm und vorzugsweise in einer Konzentration zwischen 0,01 und 5 ppm vorhanden ist.

8. Zusammensetzung nach einem der Ansprüche 6 oder 7, **dadurch gekennzeichnet, dass** sie außerdem Glycerol als Feuchthaltemittel enthält.

9. Verwendung einer wirksamen Menge von Peptid, alleine oder zusammen mit einem anderen Wirkstoff, für die Zubereitung einer kosmetischen oder nutrazeutischen Zusammensetzung, dazu ausgelegt, die Hydrierung und die Barrierefunktion der Epidermis zu verbessern, wobei das Peptid dazu in der Lage ist, die Synthese von Proteinen der Familie der Aquaporine zu aktivieren und die folgende allgemeine Formel (I) aufweist:
R₁-(AA)ₙ-X₁-X₂-X₃-Pro-X₄-X₅-X₆-(AA)ₚ-R₂
wobei:
X₁ Serin oder keine Aminosäure ist,
X₂ Methionin oder Leucin oder keine Aminosäure ist,
X₃ Asparagin oder Glutamin oder keine Aminosäure ist,
X₄ Alanin oder Glycin ist,
X₅ Arginin oder Lysin oder keine Aminosäure ist,
X₆ Serin, Asparaginsäure oder keine Aminosäure ist,
AA irgendeine Aminosäure darstellt oder eines ihrer Derivate, und n und p ganze Zahlen zwischen 0 und 4 sind,
R₁ die primäre Aminofunktion der N-terminalen Aminosäure darstellt, frei oder substituiert durch eine Schutzgruppe, die ausgewählt sein kann aus einer Acetylgruppe, einer Benzoylgruppe, einer Tosylgruppe oder einer Benzyloxycarbonylgruppe,
R₂ die Hydroxylgruppe der Carboxylfunktion der C-terminalen Aminosäure darstellt, frei oder substituiert durch eine Schutzgruppe, die aus einer Alkylkette von C₁ bis C₂₀ oder einer NH2-, NHY- oder NYY-Gruppe gewählt werden kann, wobei Y eine Alkylkette von C₁ bis C₄ darstellt,
wobei die Sequenz mit der allgemeinen Formel (I) aus 2 bis 15 Rückständen von Aminosäuren besteht,
wobei die Sequenz mit der allgemeinen Formel (I) Substitutionen der Aminosäuren X₁ bis X₆ durch andere chemisch gleichwertige Aminosäuren umfassen kann.

10. Verwendung einer wirksamen Menge von Peptid, wie in Anspruch 9 definiert, als Wirkstoff, alleine oder zusammen mit einem anderen Wirkstoff, für die Zubereitung einer kosmetischen oder nutrazeutischen Zusammensetzung, dazu ausgelegt, Hauttrockenheit vorherzusagen oder zu behandeln.

11. Verwendung einer wirksamen Menge von Peptid, wie in Anspruch 9 definiert, als Wirkstoff, alleine oder zusammen mit einem anderen Wirkstoff, für die Zubereitung einer kosmetischen oder nutrazeutischen Zusammensetzung, dazu ausgelegt, die Haut und die Schleimhäute vor äußeren Aggressionen zu schützen.

12. Verwendung einer wirksamen Menge von Peptid, wie in Anspruch 9 definiert, als Wirkstoff, alleine oder zusammen mit einem anderen Wirkstoff, für die Zubereitung einer kosmetischen oder nutrazeutischen oder pharmazeutischen Zusammensetzung, dazu ausgelegt, die Regeneration der Haut zu stimulieren.

13. Verwendung einer wirksamen Menge von Peptid, wie in Anspruch 9 definiert, als Wirkstoff, alleine oder zusammen mit einem anderen Wirkstoff, für die Zubereitung einer kosmetischen oder nutrazeutischen Zusammensetzung, dazu ausgelegt, die Hautanzeichen der Alterung und/oder der Lichtalterung vorzubeugen oder zu behandeln.

14. Verwendung einer wirksamen Menge von Peptid, wie in Anspruch 9 definiert, als Wirkstoff, alleine oder zusammen mit einem anderen Wirkstoff, für die Zubereitung einer pharmazeutischen Zusammensetzung, dazu ausgelegt, die pathologischen Zustände, verbunden mit der Trockenheit der Haut oder der Schleimhäute, zu behandeln.

15. Verfahren zur kosmetischen Behandlung, dazu ausgelegt, das Aussehen der Haut zu verbessern die Trockenheit der Haut und der Schleimhäute vorzubeugen und/oder dagegen zu kämpfen, **dadurch gekennzeichnet, dass** topisch auf der zu behandelnden Haut oder auf den zu behandelnden Schleimhäuten eine Zusammensetzung angewendet wird, wie nach einem der Ansprüche 6 bis 8 definiert.

16. Verfahren zur kosmetischen Behandlung, dazu ausgelegt, die Hautanzeichen der Alterung und/oder der Lichtalterung vorzubeugen und/oder dagegen zu kämpfen, **dadurch gekennzeichnet, dass** topisch auf der zu behandelnden Haut eine Zusammensetzung angewendet wird, wie nach einem der Ansprüche 6 bis 8 definiert.

## Claims

1. Peptide capable of activating the synthesis of proteins in the aquaporins family, **characterised in that** it corresponds to one of the following sequences:
(SEQ ID No. 1) Ser-Met-Asn-Pro-Gly-Arg
(SEQ ID No. 2) Gln-Pro-Ala-Lys-Ser
(SEQ ID No. 3) Asn-Pro-Ala-Arg-Asp
(SEQ ID No. 4) Asn-Pro-Ala-Arg
(SEQ ID No. 5) Asn-Pro-Ala-Arg-NH₂
(SEQ ID No. 6) Asn-Pro-Ala
(SEQ ID No. 7) Asn-Pro-Ala-NH₂
(SEQ ID No. 9) Pro-Ala-Arg-NH₂

2. Peptide according to claim 1, **characterised in that** it corresponds to sequence SEQ ID No. 4.

3. Peptide according to claim 1, **characterised in that** it corresponds to sequence SEQ ID No. 5.

4. Peptide according to any one of the previous claims, **characterised in that** it has at least one functional group protected by a protective group, this protective group being either an acylation or an acetylation of the amino-terminal end, or an amidation or an esterification of the carboxy-terminal end, or both.

5. Peptide according to any one of the previous claims, **characterised in that** the proteins in the aquaporins family are type 3 aquaporins.

6. Cosmetic or pharmaceutical composition **characterised in that** it contains an effective quantity of a peptide in a physiologically acceptable medium as an active constituent, capable of activating the synthesis of proteins in the aquaporins family and corresponding to one of the following sequences:
(SEQ ID No. 1) Ser-Met-Asn-Pro-Gly-Arg
(SEQ ID No. 2) Gln-Pro-Ala-Lys-Ser
(SEQ ID No. 3) Asn-Pro-Ala-Arg-Asp
(SEQ ID No. 4) Asn-Pro-Ala-Arg
(SEQ ID No. 5) Asn-Pro-Ala-Arg-NH₂
(SEQ ID No. 6) Asn-Pro-Ala
(SEQ ID No. 7) Asn-Pro-Ala-NH₂
(SEQ ID No. 8) Pro-Ala-Arg
(SEQ ID No. 9) Pro-Ala-Arg-NH₂
previously solubilised in one or several cosmetically or pharmaceutically acceptable solvents such as water, glycerol, ethanol, propylene glycol, butylene glycol, dipropylene glycol, ethoxylated or propoxylated diglycols, cyclic polyols, vaseline, a vegetable oil or any mix of these solvents.

7. Cosmetic or pharmaceutical composition according to claim 6, **characterised in that** said peptide is present at a concentration of between approximately 0.0005 and 500 ppm, and preferably at a concentration of between 0.01 and 5 ppm.

8. Composition according to any one of claims 6 or 7, **characterised in that** it also contains glycerol as a moisturising agent.

9. Use of an efficient quantity of peptide alone or in association with another active constituent for the preparation of a cosmetic or nutraceutic composition intended to improve moisturisation and the barrier function of the epidermis, said peptide being capable of activating the synthesis of proteins in the aquaporins family and generally with the following formula:
R₁-(AA)n-X₁-X₂-X₃-Pro-X₄-X₅-X₆-(AA)ₚ-R2
in which,
X₁ is serine or no amino acid
X₂ is methionine or leucine or no amino acid
X₃ is asparagine or glutamine or no amino acid
X₄ is alanine or glycine
X₅ is arginine or lysine or no amino acid
X₆ is serine, aspartic acid or no amino acid,
AA represents any amino acid or one of its derivatives, and n and p are integer numbers between 0 and 4,
R₁ represents the primary amine function of the N-terminal amino acid, free or substituted by a protective group that can be chosen from among an acetyl group, a benzoyl group, a tosyl group or a benzyloxycarbonyl group,
R₂ represents the hydroxyl group of the carboxyl function of C-terminal amino acid, free or substituted by a protective group that can be chosen from among an alkyl chain from C₁ to C₂₀, or an NH2, NHY or NYY group where Y represents an alkyl chain from C₁ to C₄.
said sequence with general formula (I) being composed of 2 to 15 residues of amino acids,
said sequence with general formula (I) possibly comprising substitutions of amino acids X₁ to X₆ by other chemically equivalent amino acids.

10. Use of an effective quantity of peptide as defined in claim 9 as active constituent, alone or in association with another active constituent, for the preparation of a cosmetic or nutraceutic composition intended to prevent or treat skin dryness.

11. Use of an effective quantity of peptide as defined in claim 9 as active constituent, alone or in association with another active constituent, for the preparation of a cosmetic or nutraceutic composition intended to protect the skin and mucous membranes from external aggression.

12. Use of an effective quantity of peptide as defined in claim 9 as active constituent, alone or in association with another active constituent, for the preparation of a cosmetic or nutraceutic or pharmaceutical composition intended to stimulate skin regeneration.

13. Use of an effective quantity of peptide as defined in claim 9 as active constituent, alone or in association with another active constituent, for the preparation of a cosmetic or nutraceutic composition intended to prevent or treat signs of skin aging and/or photo-aging.

14. Use of an efficient quantity of peptide as defined in claim 9 as active constituent, either alone or in association with another active constituent, to prepare a pharmaceutical composition intended to treat pathological conditions related to dryness of the skin or mucous membranes.

15. Cosmetic treatment method intended to improve appearance of the skin and to prevent and/or reduce dryness of the skin and mucous membranes, **characterised in that** a composition like that defined in any one of claims 6 to 8 is applied topically on the skin or mucous membranes to be treated.

16. Cosmetic treatment method intended to prevent and/or reduce signs of skin aging and/or photo-aging, **characterised in that** a composition like that defined in any one of claims 6 to 8 is applied topically on the skin to be treated.


### SEQUENCE LISTING

<110> société d'Extraction des Principes Actifs (Vincience)
<120> PEPTIDE ACTIVATEUR DE LA SYNTHESE DES AQUAPORINES ET COMPOSITION COSMETIQUE ET/OU PHARMACEUTIQUE LE CONTENANT
<130> BV PCT 07-97
<150> FR0708977
   <151> 2007-12-21
<160> 10
<170> PatentIn version 3.3
<210> 1
   <211> 6
   <212> PRT
   <213> Artificial
<220>
   <223> Synthetic peptide
<400> 1
   Ser Met Asn Pro Gly Arg
   1               5
<210> 2
   <211> 5
   <212> PRT
   <213> Artificial
<220>
   <223> Synthetic peptide
<400> 2
   Gln Pro Ala Lys Ser
   1 5
<210> 3
   <211> 5
   <212> PRT
   <213> Artificial
<220>
   <223> Synthetic peptide
<400> 3
   Asn Pro Ala Arg Asp
   1 5
<210> 4
   <211> 4
   <212> PRT
   <213> Artificial
<220>
   <223> synthetic peptide
<400> 4
   Asn Pro Ala Arg
   1
<210> 5
   <211> 4
   <212> PRT
   <213> Artificial
<220>
   <223> Synthetic peptide
<220>
   <221> MOD_RES
   <222> (4)..(4)
   <223> AMIDATION
<400> 5
   Asn Pro Ala Arg
   1
<210> 6
   <211> 3
   <212> PRT
   <213> Artificial
<220>
   <223> Synthetic peptide
<400> 6
   Asn Pro Ala
   1
<210> 7
   <211> 3
   <212> PRT
   <213> Artificial
<220>
   <223> Synthetic peptide
<220>
   <221> MOD_RES
   <222> (3)..(3)
   <223> AMIDATION
<400> 7
   Asn Pro Ala
   1
<210> 8
   <211> 3
   <212> PRT
   <213> Artificial
<220>
   <223> Synthetic peptide
<400> 8
   Pro Ala Arg
   1
<210> 9
   <211> 3
   <212> PRT
   <213> Artificial
<220>
   <223> Synthetic peptide
<220>
   <221> MOD_RES
   <222> (3)..(3)
   <223> AMIDATION
<400> 9
   Pro Ala Arg
   1
<210> 10
   <211> 4
   <212> PRT
   <213> Artificial
<220>
   <223> synthetic peptide
<400> 10
   Leu Asn Pro Ala
   1